# FASCICULE DE BREVET EUROPEEN

(11) **EP 1 537 216 B1**
(45) Date de publication et mention de la délivrance du brevet: **09.11.2011**
(21) Numéro de dépôt: 03769603.6
(22) Date de dépôt: 10.09.2003
(51) Int. Cl.: C12N 15/82, A01H 5/00

(54) **PLANTES TRANSFORMEES A BIOSYNTHESE DE PRENYLQUINONES AMELIOREE**
TRANSFORMIERTE PFLANZEN MIT VERBESSERTER BIOSYNTHESE VON PRENYLQUINONEN
TRANSFORMED PLANTS WITH ENHANCED PRENYLQUINONE BIOSYNTHESIS

(30) Priorité: 11.09.2002 FR 0211209
(43) Date de publication de la demande: 08.06.2005
(73) Titulaire: Bayer S.A.S., 69009 Lyon (FR)
(72) Inventeur: MATRINGE, Michel, F-38100 Grenoble (FR); RIPPERT, Pascal, CH-8044 Zürich (CH); DUBALD, Manuel, F-69370 Saint Didier au Mont d'Or (FR); DUMAS, Renaud, F-38300 Bourgoin-Jallieu (FR)
(74) Mandataire: Monconduit, Hervé
(86) Numéro de dépôt international: PCT/FR2003/002684
(87) Numéro de publication internationale: WO 2004/024928

(56) Documents cités:
- WO-A-00/08169
- WO-A-00/61771
- WO-A-02/46441
- WO-A-96/38567
- WO-A-99/23231
- WO-A-02/089561
- MANNHAUPT G ET AL: "CHARACTERIZATION OF THE PREPHENATE DEHYDROGENASE-ENCODING GENE TYR1 FROM SACCHAROMYCES-CEREVISIAE" GENE (AMSTERDAM), vol. 85, no. 2, 1989, pages 303-312, XP002242863 ISSN: 0378-1119 cité dans la demande
- GARCIA ISABELLE ET AL: "Characterization and subcellular compartmentation of recombinant 4-hydroxyphenylpyruvate dioxygenase from Arabidopsis in transgenic tobacco." PLANT PHYSIOLOGY (ROCKVILLE), vol. 119, no. 4, avril 1999 (1999-04), pages 1507-1516, XP002242864 ISSN: 0032-0889 cité dans la demande

## Description

La présente invention concerne des plantes transformées, en particulier des plantes transformées produisant des quantités plus importantes de plastoquinones, tocotriénols et tocophérols, que des plantes identiques non transformées. Cette invention concerne également un procédé de production de ces plantes, ainsi qu'un procédé de culture de ces plantes. Les plantes selon l'invention ont également la propriété d'être tolérantes aux herbicides inhibiteurs de l'enzyme p-hydroxyphenylpyruvate dioxygénase (ci-après dénommée HPPD).

Les prénylquinones sont un large groupe de composés à affinités lipidiques comprenant, entre autres, les plastoquinones, les tocophérols, et les tocotriénols. Chez les plantes, les prénylquinones sont synthétisés via la voie de l'homogentisate.

La plus connue de prénylquinones est la vitamine E, ou α-tocophérol, élément essentiel de l'alimentation humaine et animale, en particulier celle des mammifères qui n'en produisent pas naturellement, mais en ont un besoin alimentaire. L'effet le plus reconnu de la vitamine E est son action antioxydante sur les lipides des membranes cellulaires (Epstein et al., 1966, Radical Research 28: 322-335; Kamel-Eldin et Appelqvist, 1996, Lipids 31: 671-701).

Au-delà de la vitamine E, il a été démontré que les tocotriénols, bien que non essentiels dans l'alimentation humaine et animale, possèdent des propriétés anti-oxydantes particulièrement intéressantes, plus élevées que celles de la vitamine E (Kamat et al., 1997, Mol. Cell. Biochem. 170, 131-137). Ces composés sont notamment connus pour protéger les cellules contre les radicaux libres, ainsi que pour prévenir l'apparition de maladies cardio-vasculaires ou de cancers (Packer et al., 2001, J. Nutr. 131(2): 369S-373S). En outre, les tocotriénols présentent une activité anti-cancer par inhibition de la prolifération de récepteurs estrogènes, activité que ne possèdent pas les tocophérols (Guthrie et al., 1997, J. Nutr. 127: 544-548). Ils présentent également une bien meilleure activité hypocholestérolémique que les tocophérols, (Pearce et al., 1992, J. Med. Chem. 35: 3595-3606; Qureshi et al., 2001, J. Nutr. 131: 2606-2618), ce qui les rend plus aptes à lutter contre l'artériosclérose.

Les plastoquinones n'ont pas de rôle connu en santé humaine ou animale, mais jouent un rôle capital dans les plantes. Ces molécules sont présentes dans les membranes des chloroplastes et ont pour fonction le transport d'électrons au cours de la réaction de photosynthèse (Grumbach, 1984, Structure Function and Metabolisme of plant lipids, Siegenthaler and Eichenberger eds).

En outre, une augmentation de la quantité de prénylquinones devrait conférer aux plantes une meilleure résistance vis-à-vis des stress oxydants, en particulier le froid, la sécheresse, ou une forte illumination.

Chez les plantes et les organismes photosynthétiques en général, l'homogentisate constitue le précurseur aromatique des prénylquinones. L'homogentisate est le produit de l'enzyme p-hydroxyphenylpyruvate dioxygénase (ci-après dénommée HPPD). Chez la plupart des organismes, les HPPD sont des enzymes impliquées dans la voie de dégradation catabolique de l'acide aminé aromatique Tyrosine (Goodwin, 1972, in Tyrosine Metabolism: The biochemical, physiological, and clinical significance of p-hydroxyphenylpyruvate oxygenase, Goodwin B.L., ed., Oxford University press, 1-94). Les HPPD catalysent la réaction de transformation du para-hydroxy-phényl-pyruvate (HPP), produit de dégradation de la tyrosine, en homogentisate.

La plupart des plantes synthétisent la tyrosine *via* l'arogénate (Abou-Zeid et al. 1995 Applied Env Microb 41: 1298-1302,; Bonner et al., 1995 Plant Cells Physiol. 36, 1013-1022; Byng et al., 1981 Phytochemistry 6: 1289-1292; Connely and Conn 1986 Z. Naturforsch 41c: 69-78; Gaines et al., 1982 Planta 156: 233-240). Chez ces plantes, l'HPP dérive uniquement de la dégradation de la tyrosine. Par contre, chez des organismes comme la levure *Sacharomyces cerevisiae* ou la bactérie *Escherichia coli,* l'HPP est un précurseur de la Tyrosine, et il est synthétisé par l'action d'une enzyme préphénate déshydrogénase (ci-après PDH) qui transforme le préphénate en HPP (Lingens et al., 1967 European J. Biochem 1: 363-374; Sampathkumar and Morrisson 1982 Bioch Biophys Acta 702: 204-211). Chez ces organismes, la production d'HPP est donc reliée directement à la voie de biosynthèse des acides aminés aromatiques (voie du shikimate), et non pas à la voie de dégradation de la tyrosine (voir Figure 1).

Afin d'augmenter la biosynthèse de prénylquinones par les plantes, les inventeurs de la présente fascicule de brevet ont cherché à augmenter le flux du précurseur HPP dans les cellules de ces plantes en connectant la synthèse dudit précurseur à la voie dite du shikimate par surexpression d'une enzyme PDH. L'effet attendu est un flux plus important du précurseur HPP devant augmenter globalement la biosynthèse de prénylquinones.

Il a effectivement été constaté que la transformation de plantes avec un gène codant pour une enzyme PDH permettait d'augmenter la production de prénylquinones par lesdites plantes. Cette augmentation est très significative lorsque les plantes transformées avec un gène codant une enzyme PDH sont des plantes sur-exprimant également une enzyme HPPD. Un constat similaire est réalisé dans WO 02/089561 avec l'enzyme PDH codée par le gène TyrA d'*Erwinia herbicola.*

Il a également été constaté que la transformation de plantes avec un gène codant pour une enzyme PDH permettait d'augmenter la tolérance desdites plantes aux inhibiteurs d'HPPD. Cette augmentation de tolérance est très significative lorsque les plantes transformées avec un gène codant une enzyme PDH sont des plantes sur-exprimant également une enzyme HPPD.

Depuis quelques années, l'intérêt pour les HPPD s'est considérablement accru suite à la démonstration que cette enzyme est la cible de nouvelles familles d'herbicides dit "blanchissants". De tels herbicides ayant pour cible l'HPPD sont notamment les isoxazoles (EP 418 175, EP 470 856, EP 487 352, EP 527 036, EP 560 482, EP 682 659, US 5 424 276) en particulier l'isoxaflutole, herbicide sélectif du maïs, les dicétonitriles (EP 496 630, EP 496 631), en particulier la 2-cyano-3-cyclopropyl-1-(2-SO₂ CH₃-4-CF₃ phényl) propane-1,3-dione et la 2-cyano-3-cyclopropyl-1-(2-SO₂ CH₃-4-2,3 Cl₂ phényl) propane-1, 3-dione, les tricétones (EP 625 505, EP 625 508, US 5,506,195), en particulier la sulcotrione ou la mésotrione, ou encore les pyrazolinates.

Un des avantages des herbicides ayant pour cible des enzymes impliquées dans les voies métaboliques vitales des plantes est leur large spectre d'activité sur des plantes d'origines phylogénétiques éloignées. Toutefois, de tels herbicides présentent également l'inconvénient majeur, lorsqu'ils sont appliqués sur des cultures pour éliminer les végétaux indésirables ou "mauvaises herbes", d'agir également sur les plantes cultivées. Cet inconvénient peut être pallié par l'utilisation de plantes cultivées tolérantes aux dits herbicides. De telles plantes sont généralement obtenues par génie génétique en introduisant dans leur génome un gène codant une enzyme de résistance audit herbicide de manière à ce qu'elles surexpriment ladite enzyme dans leurs tissus.

Jusqu'à présent trois principales stratégies utilisant le génie génétique ont été employée pour rendre des plantes tolérantes aux herbicides. La première consiste à détoxifier l'herbicide par transformation de la plante avec un gène codant une enzyme de détoxification. Cette enzyme transforme l'herbicide, ou son métabolite actif, en produits de dégradation non toxique, comme par exemple les enzymes de tolérance au bromoxynil ou au basta (EP 242 236, EP 337 899). La seconde stratégie consiste à transformer la plante avec un gène codant l'enzyme cible mutée de manière à ce qu'elle soit moins sensible à l'herbicide, ou son métabolite actif, comme par exemple les enzymes de tolérance au glyphosate (EP 293 356; Padgette et al., 1991, J. Biol. Chem. 266: 33). La troisième stratégie consiste à surexprimer l'enzyme cible sensible, de manière à produire dans la plante des quantités importantes d'enzyme cible, si possible bien supérieures à la quantité d'herbicide pénétrant la plante. Cette stratégie permet de maintenir un niveau suffisant d'enzyme fonctionnelle, malgré la présence de son inhibiteur.

Cette troisième stratégie a été mise en oeuvre et a permis d'obtenir des plantes tolérantes aux inhibiteurs d'HPPD (WO 96/38567). En outre, cette stratégie de simple surexpression de l'enzyme cible sensible (non mutée) était employée pour la première fois avec succès pour conférer aux plantes une tolérance à un niveau agronomique à un herbicide.

On sait également que la plupart des herbicides inhibiteurs de l'HPPD sont des inhibiteurs compétitifs vis à vis du substrat, à fixation lente et quasi irréversible (Ellis et al., 1996, Chem. Res. Toxicol. 9: 24-27; Viviani et al., 1998, Pestic. Biochem. Physiol. 62: 125-134). Leur mode d'action consiste donc à entrer en compétition avec l'HPP en se fixant de manière préférentielle sur son site de fixation. Le résultat de cette fixation est l'arrêt de synthèse par la cellule de l'homogentisate.

La présente invention, mettant en oeuvre une augmentation du flux du substrat HPP de l'HPPD par sur-expression d'une enzyme PDH, semble constituer une quatrième stratégie possible pour obtenir de plantes tolérantes aux herbicides, en particulier aux herbicides inhibiteurs de l'HPPD.

La présente invention concerne donc des plantes transformées, caractérisées en ce qu'elles comprennent:
(1) un gène chimère comprenant un promoteur fonctionnel dans les plantes et une séquence codant pour une enzyme Préphénate Deshydrogénase (PDH), à l'exception de la séquence codante du gène TyrA *d'Erwinia herbicola*
(2) un gène chimère comprenant un promoteur fonctionnel dans les plantes et une séquence codant pour une enzyme p-hydroxyphenylpyruvate dioxygénase (HPPD)

Selon un mode particulier de réalisation de l'invention, les plantes transformées selon l'invention peuvent être représentées par des cellules végétales transformées.

Par plantes transformées ou cellules végétales transformées, on entend des plantes ou des cellules végétales ayant intégré de manière stable dans leur génome au moins un transgène, ledit transgène pouvant provenir de la plante transformée ou de tout autre organisme. De préférence, un transgène est représenté par un gène chimère comprenant des éléments provenant d'au moins un autre organisme que la plante transformée. En particulier, un transgène peut contenir, entre autres éléments, au moins un promoteur, une séquence codante et un terminateur provenant d'organismes différents, lesdits organismes étant également différents de la plante transformée.

Le terme PDH doit être interprété comme faisant référence à toute enzyme PDH native, ou mutée, présentant l'activité PDH de transformation du préphénate en HPP. En particulier, ladite enzyme PDH peut provenir de tout type d'organisme. L'identification d'une enzyme à activité PDH peut être réalisée par toute méthode permettant soit de mesurer la diminution de la quantité du substrat préphénate, soit de mesurer l'accumulation d'un produit issu de la réaction enzymatique, à savoir l'HPP ou un des cofacteurs NADH ou NADPH. En particulier, la mesure de l'activité PDH peut être réalisée par la méthode décrite à l'exemple 2.

De nombreux gènes codant des enzymes PDH sont décrites dans la littérature et leurs séquences peuvent être identifiées sur le site Web http://www.ncbi.nlm.nih.gov/entrez/. On connaît notamment le gène codant l'enzyme PDH de la levure *Saccharomyces cerevisiae* (No. d'accession S46037) telle que décrite dans Mannhaupt et al. (1989, Gene 85, 303-311), de bactérie du genre *Bacillus,* en particulier de l'espèce *B. subtilis* (No. d'accession P20692) telle que décrite dans Henner et al. (1986, Gene 49 (1), 147-152), de bactérie du genre *Escherichia,* en particulier de l'espèce *E. coli* (No. d'accession KMECTD) telle que décrite dans Hudson et al. (1984, J. Mol. Biol. 180(4), 1023-1051), ou de bactérie du genre *Erwinia,* en particulier de l'espèce d'E. *herbicola* (No. d'accession S29934) telle que décrite dans Xia et al. (1992, J. Gen. Microbiol. 138(7), 1309-1316).

Le terme HPPD doit être interprété comme faisant référence à toute enzyme HPPD native, mutée ou chimère, présentant l'activité HPPD de transformation de l'HPP en homogentisate. Une mesure de l'activité enzymatique des HPPD peut être réalisée par toute méthode permettant soit de mesurer la diminution de la quantité du substrat HPP, soit de mesurer l'accumulation du produit issu de la réaction enzymatique, à savoir l'homogentisate. En particulier, la mesure de l'activité HPPD peut être réalisée par la méthode décrite à l'exemple 1 et dans Garcia et al. (1997, Biochem. J. 325, 761-769) ou Garcia et al. (1999, Plant Physiol. 119, 1507-1516).

En particulier, ladite enzyme HPPD peut provenir de tout type d'organisme. De nombreux gènes codant des enzymes HPPD sont décrits dans la littérature, notamment les gènes de bactéries comme *Pseudomonas* (Rüetschi & al., 1992, Eur. J. Biochem., 205, 459-466, WO 96/38567), de plantes comme d'*Arabidopsis* (WO 96/38567, Genebank AF047834) ou de carotte (WO 96/38567, Genebank 87257), de *Coccicoides* (Genebank COITRP), ou de mammifères comme la souris ou le cochon.

Par HPPD mutée, on entend une HPPD possédant au moins une mutation par rapport à une HPPD native, et possédant la propriété d'être plus tolérante aux herbicides inhibiteurs d'HPPD que l'HPPD native correspondante. De manière avantageuse, l'HPPD mutée est une HPPD mutée dans sa partie C-terminale telle que décrite dans la demande de brevet WO 99/24585. De manière préférée, l'HPPD mutée comprend la mutation W336 telle que décrite dans la demande de brevet WO 99/24585.

Par HPPD chimère, on entend une HPPD comprenant des éléments provenant de différentes HPPD. De telles HPPD chimères sont notamment décrites dans la demande de brevet WO 99/24586.

De manière avantageuse, l'HPPD est une HPPD de *Pseudomonas fluorescens* (WO 96/38567) ou d'*Arabidopsis thaliana* (WO 96/38567).

Dans l'expression "gène fonctionnel dans les plantes permettant la surexpression d'une enzyme phytylprényl transférase", le terme phytylprényl transférase doit être interprété comme faisant référence à une enzyme phytylprényl transférase telle que décrite dans la demande de brevet WO 02/089561. En particulier, ledit "gène fonctionnel dans les plantes permettant la surexpression d'une enzyme phytylprényl transférase" consiste en un gène sélectionné parmi le gène slr1736 de *Synechocystis* (séquence décrite dans la Cyanobase sur le site Web http://www.kazusa.or.jp/cyanobase), et le gène ATPT2 *d'Arabidopsis* (Smith et al., 1997, Plant J. 11, 83-92).

Les plantes ou cellules végétales transformées selon l'invention produisent des quantités de prénylquinones supérieures à celles de plantes non transformées. De manière préférée, les plantes ou cellules végétales transformées selon l'invention produisent des quantités de prénylquinones supérieures à celles de plantes transformées avec un seul des gènes fonctionnel dans les plantes permettant la surexpression d'une enzyme PDH ou HPPD. De préférence, les prénylquinones produites par les plantes ou cellules végétales transformées selon l'invention sont des tocophérols et/ou des tocotriénols et/ou des plastoquinones. De nombreuses méthodes de mesure de la quantité de tocophérols, tocotriénols et plastoquinones sont connues et à la disposition de l'homme du métier. A titre d'exemple, les tocophérols, les tocotriénols, et les plastoquinones peuvent être mesurés par la méthode de Frazer et al. (2000, Plant J. 24: 551-558). Par quantités supérieures, on entend selon la présente invention des quantités de préférence au moins 2 fois supérieures, de préférence au moins 5 fois supérieures, de préférence au moins 10 fois supérieures, de préférence au moins 50 fois supérieures, de préférence au moins 100 fois supérieures, de préférence au moins 500 fois supérieures, et de manière préférée au moins 1000 fois supérieures.

Les plantes transformées selon l'invention ont également pour effet d'être tolérantes aux inhibiteurs de l'HPPD.

Par plantes transformées tolérantes aux inhibiteurs de l'HPPD, on entend des plantes transformées telles que décrites précédemment présentant au moins la caractéristique d'être tolérantes vis-à-vis d'une dose d'inhibiteur de l'HPPD normalement toxique pour des plantes identiques non transformées. La dose d'inhibiteur de l'HPPD normalement toxique pour une plante non transformée dépend de l'inhibiteur d'HPPD utilisé et de la plante sur laquelle ledit inhibiteur est appliqué, ainsi que du stade auquel il est appliqué sur ladite plante. Toutefois, un homme du métier saura déterminer une telle dose sachant que le caractère toxique dudit inhibiteur peut correspondre soit à un effet mortel dudit inhibiteur conduisant à la mort de la plante un certain nombre de jours après l'application dudit inhibiteur, ledit effet mortel pouvant être précédé par un effet dit de "blanchissement" de la plante comme cela est généralement le cas pour les inhibiteurs de l'HPPD, soit à un effet de diminution de la croissance de la plante. De préférence, les plantes transformées tolérantes aux inhibiteurs de l'HPPD selon l'invention sont tolérantes vis-à-vis d'une dose d'inhibiteur de l'HPPD normalement toxique pour des plantes identiques transformées avec uniquement le gène fonctionnel dans les plantes permettant la surexpression d'une enzyme HPPD.

Par inhibiteurs de l'HPPD, on entend tout composé, d'origine naturelle ou artificielle, capable de se lier à une enzyme HPPD de plante de manière à bloquer, de manière transitoire ou permanente, son activité enzymatique naturelle de transformation de l'HPP en homogentisate. Par le biais de cette propriété, les inhibiteurs de l'HPPD induisent la mort ou l'inhibition de croissance des plantes sur lesquelles ils sont appliqués, ladite mort intervenant généralement après un "blanchissement" desdites plantes.

A titre d'exemples d'inhibiteurs d'HPPD, on peut citer les isoxazoles (EP 418 175, EP 470 856, EP 487 352, EP 527 036, EP 560 482, EP 682 659, US 5 424 276) en particulier l'isoxaflutole, herbicide sélectif du maïs, les dicétonitriles (ci-après nommés DKN, et décrits dans EP 496 630, EP 496 631), en particulier la 2-cyano-3-cyclopropyl-1-(2-SO₂ CH₃-4-CF₃ phényl) propane-1,3-dione et la 2-cyano-3-cyclopropyl-1-(2-SO₂ CH₃-4-2,3 Cl₂ phényl) propane-1, 3-dione, les tricétones (EP 625 505, EP 625 508, US 5,506,195), en particulier la sulcotrione ou la mésotrione, ou encore les pyrazolinates.

Par gène fonctionnel dans les plantes, on entend un gène capable de fonctionner dans une plante. Un gène capable de fonctionner dans une plante est un gène capable d'exprimer la protéine pour laquelle il code dans au moins un tissu de ladite plante. En particulier, les gènes fonctionnels dans les plantes permettent la surexpression des enzymes PDH et HPPD. La surexpression d'une protéine signifie l'expression de cette protéine dans les tissus de la plante transformée à un niveau supérieur à celui existant dans une plante identique non transformée, ledit niveau étant mesuré à un stade identique de développement desdites plantes. De préférence, le gène fonctionnel dans les plantes est un gène chimère qui peut comprendre des éléments provenant d'organismes autres que la plante dans lequel il est introduit.

Les gènes fonctionnels dans les plantes sont de préférence des gènes chimères comprenant au moins, liés entre eux de manière opérationnelle, un promoteur fonctionnel dans une plante, une séquence codant pour une enzyme PDH et/ou HPPD, et un élément terminateur fonctionnel dans cette même plante. Les différents éléments qu'un gène chimère peut contenir sont, d'une part, des éléments régulateurs de la transcription, de la traduction et de la maturation des protéines, tels qu'un promoteur, une séquence codant pour un peptide signal ou un peptide de transit, ou un élément terminateur constituant un signal de polyadénylation, et d'autre part une séquence codant pour une protéine. L'expression "liés entre eux de manière opérationnelle" signifie que lesdits éléments du gène chimère sont liés entre eux de manière à ce que leur fonctionnement soit coordonné et permette l'expression de la séquence codante. A titre d'exemple, un promoteur est lié de manière opérationnelle à une séquence codante lorsqu'il est capable d'assurer l'expression de ladite séquence codante. La construction d'un gène chimère et l'assemblage de ses différents éléments est réalisable par l'emploi de techniques bien connues de l'homme du métier, notamment celles décrites dans Sambrook et al. (1989, Molecular Cloning : A Laboratory Manual, Nolan C. ed., New York: Cold Spring Harbor Laboratory Press). Le choix des éléments régulateurs constituant le gène chimère est essentiellement fonction de la plante dans laquelle ils doivent fonctionner, et l'homme du métier est capable de sélectionner des éléments régulateurs fonctionnels dans une plante donnée.

Les promoteurs que peut contenir le gène chimère selon l'invention peuvent être constitutifs, inductibles, régulés spatialement ou temporellement.

Parmi les promoteurs constitutifs qui peuvent être utilisés dans le gène chimère de la présente invention, nous pouvons citer à titre d'exemple, des promoteurs bactériens, comme celui du gène de l'octopine synthase ou celui du gène de la nopaline synthase (Sanders et al., 1987, Nucleic Acids Res. 15, 1543-1548), des promoteurs viraux, comme celui du gène contrôlant la transcription des ARN19S ou 35S du virus de la mosaïque du Choux-Fleur (CaMV; Lawton et al., 1987, Plant Mol. Biol. 9, 315-324; Odell et al., 1985, Nature, 313, 810-812), les promoteurs du virus de la mosaïque de la nervure du Manioc (CsVMV; tels que décrits dans la demande de brevet WO 97/48819). Parmi les promoteurs d'origine végétale on citera le promoteur du gène de la petite sous-unité de ribulose-biscarboxylase/oxygénase (RuBisCO), le promoteur d'un gène d'histone tel que décrit dans la demande EP 0 507 698, ou le promoteur d'un gène d'actine de riz (Wang et al., 1992, Mol. Cell. Biol., 12 (8) : 3399-3406; US 5,641,876).

Parmi les promoteurs inductibles qui peuvent être utilisés dans le gène chimère de la présente invention, nous pouvons citer à titre d'exemple, le promoteur du gène codant pour la protéine liant l'auxine (Schwob et al., 1993, Plant J. 4 (3) : 423-432), le promoteur du gène codant l'UDP-glucose flavonoide glycosyl-transférase (Ralston et al., 1988, Genet., 119 (1), 185-197), le promoteur du gène codant l'inhibiteur de la MIP protéinase (Cordero et al., 1994, Plant J., 6 (2) 141-150), ou le promoteur du gène codant la glycéraldéhyde-3-phosphate déshydrogénase (Martinez et al., 1989, J. Mol. Biol., 208 (4), 551-565; Quigley et al., 1989, J. Mol. Evol., 29 (5), 412-421; Kohler et al., 1995, Plant Mol. Biol., 29 (6), 1293-1298).

Parmi les promoteurs tissus-spécifiques qui peuvent être utilisés dans le gène chimère de la présente invention, nous pouvons citer à titre d'exemple, les promoteurs spécifiques de racines comme par exemple celui décrit dans la demande de brevet WO 00/29594, des promoteurs spécifiques des fleurs tels que ceux décrits dans les demandes de brevets WO 98/22593, WO 99/15679 ou WO 99/43818, ou des promoteurs spécifiques des fruits, en particulier des graines comme ceux décrits dans les demandes de brevets WO 91/13993, WO 92/17580, WO 98/45460, WO 98/45461, ou WO 99/16890.

Parmi les éléments terminateurs pouvant être utilisés dans le gène chimère de la présente invention, nous pouvons citer à titre d'exemple l'élément terminateur *nos* du gène codant la nopaline synthase d'*Agrobacterium tumefaciens* (Bevan et al., 1983, Nucleic Acids Res. 11 (2), 369-385), ou l'élément terminateur d'un gène d'histone tel que décrit dans la demande EP 0 633 317.

Le gène chimère peut également comprendre une séquence d'adressage subcellulaire, codant un peptide signal ou un peptide de transit. Une telle séquence, située en amont ou en aval de la séquence codant pour une enzyme HPPD et/ou PDH, permet de diriger ladite enzyme HPPD ou PDH de manière spécifique dans un compartiment cellulaire de l'organisme hôte. Par exemple, le gène chimère peut comprendre une séquence codant un peptide signal ou un peptide de transit permettant de diriger l'enzyme HPPD et/ou PDH vers un compartiment particulier du cytoplasme comme les mitochondries, les plastes, le réticulum endoplasmique ou les vacuoles.

Le rôle de telles séquences est notamment décrit dans le numéro 38 de le revue Plant Molecular Biology (1998) consacré en grande partie au transport des protéines dans les différents compartiments de la cellule végétale (Sorting of proteins to vacuoles in plant cells pp 127-144 ; the nuclear pore complex pp 145-162 ; protein translocation into and across the chloroplastic enveloppe membranes pp 91-207 ; multiple pathways for the targeting of thylakoid proteins in chloroplasts pp 209-221 ; mitochondrial protein import in plants pp 311-338).

Selon un mode de réalisation, le peptide de transit peut être un signal d'adressage chloroplastique ou mitochondrial, lequel est ensuite clivé dans les chloroplastes ou les mitochondries. De manière préférée, le gène chimère comprend une séquence d'adressage subcellulaire codant pour un peptide de transit adressant l'enzyme HPPD et/ou PDH dans les chloroplastes.

Les peptides de transit peuvent être soit simples, soit doubles. Les peptides de transit doubles sont éventuellement séparés par une séquence intermédiaire. A titre d'exemple, un peptide de transit préféré selon l'invention comprend, dans le sens de la transcription, une séquence codant pour un peptide de transit d'un gène végétal codant pour une enzyme à localisation plastidiale, une partie de séquence de la partie mature N-terminale d'un gène végétal codant pour une enzyme à localisation plastidiale, puis une séquence codant pour un second peptide de transit d'un gène végétal codant pour une enzyme à localisation plastidiale. De tels peptides de transit doubles sont par exemple décrits dans la demande de brevet EP 0 508 909.

Le gène chimère peut également comprendre d'autres séquences de régulation, qui sont situées entre le promoteur et la séquence codante, telles que des activateurs de transcription (*enhancer*), comme par exemple l'activateur de transcription du virus de la mosaïque du tabac (VMT) décrit dans la demande WO 87/07644, du virus etch du tabac (TEV) décrit par Carrington & Freed (1990, J. Virol. 64(4):1590-7), ou du virus de la mosaïque du « figwort » (Figwort Mosaic Virus, US 5 994 521). Le gène chimère peut aussi contenir des introns, en particulier des intron favorisant l'expression des gènes dans les plantes monocotylédones tels que l'intron 1 du gène de l'actine de riz décrit dans la demande de brevet WO 99/34005, ou l'intron adh1 de maïs.

Les plantes et les cellules végétales sont des plantes et des cellules végétales transformées. Pour obtenir les plantes et les cellules végétales transformées, l'homme du métier peut utiliser une des nombreuses méthodes de transformation des plantes connues.

De manière préférée, les plantes et les cellules végétales sont transformées avec un vecteur de clonage, d'expression et/ou de transformation comprenant un gène fonctionnel dans les plantes selon l'invention permettant la surexpression d'HPPD ou de PDH.

Les vecteurs qui peuvent être utiles sont par exemple des plasmides, des cosmides, des bactériophages ou des virus. De manière préférentielle, les vecteurs de transformation des cellules végétales ou des plantes sont des plasmides. De manière générale, les principales qualités d'un vecteur doivent être une capacité à se maintenir et à s'autorépliquer dans les cellules des plantes, notamment grâce à la présence d'une origine de réplication. Dans le but d'une transformation stable d'un organisme hôte, le vecteur peut aussi s'intégrer dans le génome. Le choix d'un tel vecteur ainsi que les techniques d'insertion dans celui-ci du gène selon l'invention sont largement décrits dans Sambrook et al. (1989, Molecular Cloning: A Laboratory Manual, Nolan C. ed., New York: Cold Spring Harbor Laboratory Press) et font partie des connaissances générales de l'homme du métier. De manière avantageuse, le vecteur utilisé contient également, en plus du gène, un autre gène codant un marqueur de sélection. Le marqueur de sélection permet de sélectionner les organismes hôtes effectivement transformés, c'est-à-dire ceux ayant incorporé le vecteur. Parmi les marqueurs de sélection utilisables, on peut citer des marqueur contenant des gènes de résistance aux antibiotiques tel que, par exemple, celui du gène de l'hygromycine phosphotransférase (Gritz et al., 1983, Gene 25:179-188), mais également des marqueurs contenant des gènes de tolérance aux herbicides tel que le gène *bar* (White et al., 1990, Nucleic Acid Res. 18(4):1062) pour la tolérance au bialaphos, le gène EPSPS (US 5,188,642) pour la tolérance au glyphosate ou encore le gène HPPD (WO 96/38567) pour la tolérance aux isoxazoles. On peut également citer des gènes codant pour des enzymes facilement identifiables comme l'enzyme GUS, des gènes codant pour des pigments ou des enzymes régulant la production de pigments dans les cellules transformées. De tels gènes marqueurs de sélection sont notamment décrits dans les demandes de brevet WO 91/02071, WO 95/06128, WO 96/38567, et WO 97/04103.

Parmi les méthodes de transformation utilisables pour obtenir des plantes transformées, une de celles-ci consiste à mettre les cellules ou tissus des plantes à transformer en présence de polyéthylène glycol (PEG) et des vecteurs décrits ci-dessus (Chang and Cohen, 1979, Mol. Gen. Genet. 168(1), 111-115; Mercenier and Chassy, 1988, Biochimie 70(4), 503-517). L'électroporation est une autre méthode qui consiste à soumettre les cellules ou tissus à transformer et les vecteurs à un champ électrique (Andreason and Evans, 1988, Biotechniques 6(7), 650-660; Shigekawa and Dower, 1989, Aust. J. Biotechnol. 3(1), 56-62). Une autre méthode consiste à injecter directement les vecteurs dans les cellules ou les tissus par micro-injection (Gordon and Ruddle, 1985, Gene 33(2), 121-136). De manière avantageuse, la méthode dite de "biolistique" pourra être utilisée. Elle consiste à bombarder des cellules ou des tissus avec des particules sur lesquelles sont adsorbés les vecteurs (Bruce et al., 1989, Proc. Natl. Acad. Sci. USA 86(24), 9692-9696; Klein et al., 1992, Biotechnology 10(3), 286-291; US Patent No. 4,945,050). De manière préférentielle, la transformation de cellules ou tissus végétaux peut se faire à l'aide de bactéries du genre *Agrobacterium,* de préférence par infection des cellules ou tissus desdites plantes par *A. tumefaciens* (Knopf, 1979, Subcell. Biochem. 6, 143-173; Shaw et al., 1983, Gene 23(3):315-330) ou *A. rhizogenes* (Bevan et Chilton, 1982, Annu. Rev. Genet. 16:357-384; Tepfer and Casse-Delbart, 1987, Microbiol. Sci. 4(1), 24-28). De manière préférentielle, la transformation de cellules ou tissus végétaux par *Agrobacterium tumefaciens* est réalisée selon le protocole décrit par Ishida et al. (1996, Nat. Biotechnol. 14(6), 745-750). L'homme du métier fera le choix de la méthode appropriée en fonction de la nature de la plante à transformer.

On entend par "partie de ces plantes", tout organe de ces plantes, qu'il soit aérien ou souterrain. Les organes aériens sont les tiges, les feuilles, et les fleurs comprenant les organes reproducteurs mâles et femelles. Les organes souterrains sont principalement les racines, mais ils peuvent également être des tubercules. Par "descendance", on entend principalement les graines contenant les embryons issus de la reproduction de ces plantes entre-elles. Par extension, le terme "descendance" s'applique à toutes les graines formées à chaque nouvelle génération issue de croisements dont au moins un des parents est une plante transformée. Une descendance peut également être obtenue par multiplication végétative desdites plantes transformées. Les graines peuvent être enrobées d'une composition agrochimique comprenant au moins un produit actif possédant une activité sélectionnée parmi les activités fongicide, herbicide, insecticide, nématicide, bactéricide ou virucide.

Les plantes transformées peuvent comprendre au moins un autre gène codant pour une protéine d'intérêt, lequel autre gêne est également introduit artificiellement dans le génome de la plante, simultanément, antérieurement ou postérieurement au gène fonctionnel dans les plantes permettant la surexpression de PDH et/ou HPPD. Parmi les gènes codant pour une protéine d'intérêt, on peut citer des gènes codant une autre enzyme de résistance à un herbicide, par exemple le gène codant pour l'enzyme *bar* (White et al., NAR 18:1062, 1990) pour la tolérance au bialaphos, ou le gène codant pour l'enzyme EPSPS (US 5,188,642; WO 97/04103) pour la tolérance au glyphosate. On peut également citer un gène codant pour une toxine insecticide, par exemple un gène codant pour une δ-endotoxine de la bactérie *Bacillus thuringiensis* (pour exemple, voir International Patent Application WO 98/40490). Peuvent également être contenus dans ces plantes d'autres gènes de résistance aux maladies, par exemple un gène codant pour l'enzyme oxalate oxydase tel que décrit dans la demande de brevet EP 0 531 498 ou le brevet US 5,866,778, ou un gène codant pour un autre peptide antibactérien et/ou antifongique tels que ceux décrits dans les demandes de brevets WO 97/30082, WO 99/24594, WO 99/02717, WO 99/53053, et WO99/91089. On peut également citer des gènes codant pour des caractères agronomiques de la plante, en particulier un gène codant pour une enzyme delta-6 désaturase tel que décrit dans les brevets US 5,552,306, US 5,614,313, et demandes de brevets WO 98/46763 et WO 98/46764, ou un gène codant pour une enzyme sérine acétyltransférase (SAT) tel que décrit dans les demandes de brevets WO 00/01833 et WO 00/36127.

Les gènes supplémentaires codant une protéine d'intérêt peuvent être intégrés au moyen d'un vecteur. Dans ce cas, le vecteur comprend le gène selon l'invention codant pour une enzyme PDH et/ou HPPD et au moins un gène codant pour un autre peptide ou protéine d'intérêt.

Ils peuvent également être intégrés au moyen d'au moins un autre vecteur comprenant ledit gène supplémentaire, selon les techniques usuelles définies ci-dessus.

Les plantes peuvent encore être obtenues par croisement de plantes, l'une portant le gène codant pour une enzyme PDH et/ou HPPD selon l'invention, l'autre portant un autre gène codant pour au moins un autre peptide ou protéine d'intérêt.

Les plantes transformées peuvent être des monocotylédones ou des dicotylédones. De préférence, ces plantes sont des plantes d'intérêt agronomique. De manière avantageuse, les plantes monocotylédones sont le blé, le maïs, le riz. De manière avantageuse, les plantes dicotylédones sont le colza, le soja, le tabac, le coton.

La présente invention concerne également un procédé de culture des plantes transformées selon l'invention, caractérisé en ce qu'il consiste à planter les graines desdites plantes transformées sur une surface d'un champ approprié pour la culture desdites plantes, à appliquer sur ladite surface dudit champ au moins une composition herbicide comprenant un inhibiteur d'HPPD, puis à récolter les plantes cultivées lorsqu'elles arrivent à la maturité souhaitée et éventuellement à séparer les graines des plantes récoltées.

La présente invention concerne également un procédé pour conférer aux plantes une tolérance aux inhibiteurs d'HPPD, caractérisé en ce que l'on transforme lesdites plantes, simultanément ou successivement, avec:
(1) un gène chimère comprenant un promoteur fonctionnel dans les plantes et une séquence codant pour une enzyme Préphénate Deshydrogénase (PDH), à l'exception de la séquence codante du gène TyrA *d'Erwinia herbicola*
(2) un gène chimère comprenant un promoteur fonctionnel dans les plantes et une séquence codant pour une enzyme p-hydroxyphenylpyruvate dioxygénase (HPPD)

La présente invention concerne également une utilisation des plantes ou cellules végétales selon l'invention pour produire des prénylquinones, en particulier des tocophérols, des tocotriénols et/ou des plastoquinones.

La présente invention concerne également un procédé pour augmenter la quantité de prénylquinones dans les plantes, caractérisé en ce que l'on transforme lesdites plantes, simultanément ou successivement, avec:
(1) un gène chimère comprenant un promoteur fonctionnel dans les plantes et une séquence codant pour une enzyme Préphénate Deshydrogénase (PDH), à l'exception de la séquence codante du gène TyrA *d'Erwinia herbicola*
(2) un gène chimère comprenant un promoteur fonctionnel dans les plantes et une séquence codant pour une enzyme p-hydroxyphenylpyruvate dioxygénase (HPPD)

La présente invention concerne également un procédé de production de prénylquinones, caractérisé en ce qu'il comprend une étape de mise en culture d'une cellule végétale ou d'une plante transformée selon l'invention dans un milieu de culture adapté à la croissance et à la multiplication de ladite cellule végétale ou de ladite plante.

Selon un mode particulier de réalisation dudit procédé, les prénylquinones produits sont préférentiellement des tocophérols représentés par la vitamine E.

Selon un mode particulier de réalisation dudit procédé, les prénylquinones produits sont préférentiellement des tocotriénols.

Selon un mode de réalisation particulier, ledit procédé de production de prénylquinones comprend une étape ultérieure d'extraction desdits prénylquinones produits par ladite cellule végétale ou par ladite plante transformée cultivée à la première étape.

Lorsque ledit procédé de production de prénylquinones est mis en oeuvre avec des cellules végétales transformées selon l'invention, lesdites cellules végétales sont cultivées dans un milieu de culture favorable à leur survie et à leur croissance. L'homme du métier saura déterminer la composition dudit milieu de culture de manière à permettre une croissance optimale desdites cellules végétales. A titre d'exemple, des méthodes et milieux de culture de cellules végétales sont décrits dans Murashige et Skoog (1962, Physiol. Plant. 15: 473-497) et dans Gamborg et al. (1968, Exptl. Cell Research, 50 : 151-159).

En outre, lorsque ledit procédé est mis en oeuvre avec des cellules végétales transformées selon l'invention, lesdits prénylquinones produits peuvent être sécrétés dans le milieu de culture ou non. Lorsque lesdits prénylquinones sont sécrétés dans le milieu de culture, l'étape d'extraction dudit procédé peut être précédée par une étape de récupération du milieu de culture par élimination desdites cellules végétales. Une telle étape de récupération du milieu de culture par élimination desdites cellules végétales peut se faire par tout moyen de séparation de fractions solides comprises dans une fraction liquide. En particulier, la filtration et la centrifugation sont des moyens adaptés à la mise en oeuvre de cette étape.

Lorsque les prénylquinones ne sont pas sécrétés dans le milieu de culture, l'étape d'extraction peut être réalisée par la succession d'étapes de concentration des cellules végétales cultivées, de cassage cellulaire des cellules végétales isolées, de centrifugation de l'extrait cellulaire cassé, puis de récupération du surnageant comprenant lesdits prénylquinones. L'étape de cassage cellulaire peut être réalisée par la mise en oeuvre de techniques connues de l'homme de l'art telles que le broyage mécanique (par différence de pression, par action des ultrasons, par trituration), la lyse enzymatique, ou le choc osmotique, lesdites techniques pouvant être employées individuellement ou en combinaison.

Lorsque ledit procédé de production de prénylquinones est mis en oeuvre avec des plantes transformées selon l'invention, lesdites plantes sont cultivées sur un substrat approprié à leur survie et à leur croissance, ledit substrat pouvant être naturel ou artificiel. Un substrat naturel peut par exemple être de la terre, ou un mélange de terres, et lesdites plantes pourront être cultivées en conditions contrôlées comme par exemple en chambre de culture, en conditions semi-contrôlées comme par exemple en serre, ou en conditions naturelles comme par exemple en plein champ. Un substrat artificiel peut par exemple être un substrat liquide ou gélosé dont la composition est favorable à la survie et à la croissance des plantes selon l'invention. L'homme du métier saura déterminer la composition dudit substrat artificiel de manière à permettre une croissance optimale desdites plantes. A titre d'exemple de substrats de culture de plantes on peut citer des milieux de type laine de Roche, ou vermiculite irrigués par une solution nutritive contenant les éléments nutritifs N (Azote), P (Phosphore), K (Potassium), ou toute autre solution nutritive, commerciale ou adaptée, permettant aux plantes de pousser sur ces milieux. Lorsque les plantes selon l'invention sont cultivées sur un substrat artificiel, elles sont généralement cultivées en conditions contrôlées en chambre de culture.

En outre, lorsque ledit procédé est mis en oeuvre avec des plantes transformées lesdits prénylquinones produits sont généralement immobilisés dans lesdites plantes transformées.

Les plantes transformées, ou une partie desdites plantes, peuvent soit être utilisées directement et incorporées dans des compositions alimentaires destinées à l'alimentation humaine ou animale, soit subir une extraction des prénylquinones qu'elle contiennent. Comme indiqué précédemment, par "partie de plantes" on entend tout organe de ces plantes, qu'il soit aérien ou souterrain. Les organes aériens sont les tiges, les feuilles, et les fleurs comprenant les organes reproducteurs mâles et femelles, ainsi que les graines. Les organes souterrains sont principalement les racines, mais ils peuvent également être des tubercules. Selon un mode préféré de réalisation de l'invention, les graines sont les parties des plantes transformées destinées à l'alimentation.

La description comprend également les graines des plantes transformées, lesdites graines étant riches en prénylquinones comparativement à des graines de plantes non transformées. En outre, la description comprend également des compositions alimentaires comprenant des graines ou toute autre partie des plantes transformées selon l'invention. L'huile produite à partir de ces parties de plantes, en particulier des graines, est également décrit.

Afin de récupérer les prénylquinones produits dans la plante transformée, une étape d'extraction peut être réalisée par la succession d'étapes de broyage des plantes cultivées, de filtration et/ou centrifugation du broyat de plantes, puis de récupération du surnageant comprenant lesdits prénylquinones, ladite récupération pouvant consister en une extraction des composés lipidiques. De préférence, l'étape de broyage consiste en un broyage mécanique (par différence de pression, par action des ultrasons, par trituration), lequel peut être suivi d'une lyse enzymatique, ou d'un choc osmotique.

Le présent procédé peut également mettre en oeuvre une étape finale de purification des prénylquinones contenus dans l'extrait de cellule végétale ou de plante obtenu. La purification desdits prénylquinones peut se faire par toute technique de concentration ou de séparation de composés, en particulier les techniques de microfiltration, d'ultrafiltration, d'électrophorèse ou de chromatographie bien connues de l'homme du métier. Afin de parvenir à des prénylquinones purifiés, l'homme du métier saura employer une méthode de mesure desdits prénylquinones pour identifier la ou les fractions de purification contenant lesdits prénylquinones. Selon ce procédé, lesdits prénylquinones produits peuvent avoir une pureté de préférence de 50%, de 60%, 70%, 80%, 90%, 95%, 99%, ou avantageusement 100%.

Selon un mode particulier de réalisation de l'invention, les plantes transformées selon l'invention comprennent, en plus d'un gène chimère comprenant un promoteur fonctionnel dans les plantes et une séquence codant pour une enzyme Préphénate Deshydrogénase (PDH), à l'exception de la séquence codante du gène TyrA *d'Erwinia herbicola* et d'un gène chimère comprenant un promoteur fonctionnel dans les plantes et une séquence codant pour une enzyme p-hydroxyphenylpyruvate dioxygénase (HPPD), un gène chimère comprenant un promoteur fonctionnel dans les plantes et une séquence codant pour une enzyme géranylgéranyl réductase (GGR). Parmi les prénylquinones produites, de telles plantes produisent préférentiellement des tocophérols, en particulier de la vitamine E, comparativement aux tocotriénols et aux plastoquinones.

L'enzyme GGR est une enzyme catalysant la transformation du géranylgéranyl pyrophosphate en phytylpyrophosphate. Selon un mode particulier de réalisation, le gène fonctionnel dans les plantes permettant la surexpression de GGR comprend la séquence codante d'un gène codant pour une GGR de plante. A titre d'exemple, on peut utiliser la séquence codant pour la GGR *d'Arabidopsis* telle que publiée dans Keller et al., (1998, Eur. J. Biochem. 251(1-2): 413-417), ou celles décrites par les numéros d'accession AJ 007789 (Tabac), AF 069318 (*Mesembryanthenum crystallinum*), Y14044 (*Arabidopsis*), Q55087 (*Synechocystis sp* PCC 6803).

### Références bibliographiques

Abou-Zeid et al., 1995, Applied Env Microb 41: 1298-1302
Andreason et Evans, 1988, Biotechniques 6(7): 650-660
Ausubel et al., 1994, Current Protocols in Molecular Biology, Current protocols, USA, Vol.1-2
Bevan et al., 1983, Nucleic Acids Res. 11 (2): 369-385
Bevan et Chilton, 1982, Annu. Rev. Genet. 16: 357-384
Bonner et al., 1995, Plant Cells Physiol. 36: 1013-1022
Brown, 1998, Molecular Biology LabFax, Second edition, Academic Press, UK
Bruce et al., 1989, Proc. Natl. Acad. Sci. USA 86(24): 9692-9696
Byng et al., 1981 Phytochemistry 6: 1289-1292
Carrington & Freed, 1990, J. Virol. 64(4):1590-1597
Chang and Cohen, 1979, Mol. Gen. Genet. 168(1): 111-115
Connely and Conn, 1986, Z. Naturforsch 41c: 69-78
Cordero et al., 1994, Plant J., 6 (2): 141-150
Croy R.D.D., 1993, Plant Molecular Biology LabFax, BIOS Scientific Publications Ltd (UK) and Blackwell Scientific Publications (UK)
Dieffenbach and Dveksler, 1995, PCR Primer: A laboratory manual, Cold Spring Harbor Laboratory Press, NY
Ellis et al., 1996, Chem. Res. Toxicol. 9: 24-27
Epstein et al., 1966, Radical Research 28: 322-335
Folch et al., 1957, J. Biol. Chem.: 226-497
Frazer et al., 2000, Plant J. 24: 551-558
Gaines et al., 1982, Planta 156: 233-240
Gamborg et al., 1968, Exptl. Cell Research, 50: 151-159
Garcia et al.,1997, Biochem. J. 325: 761-769
Garcia et al., 1999, Plant Physiol. 119: 1507-1516
Goodwin, 1972, in Tyrosine Metabolism: The biochemical, physiological, and clinical significance of p-hydroxyphenylpyruvate oxygenase, Goodwin B.L., ed., Oxford University press: 1-94
Gordon and Ruddle, 1985, Gene 33(2): 121-136
Gritz et al., 1983, Gene 25: 179-188
Grumbach, 1984, Structure Function and Metabolisme of plant lipids, Siegenthaler and Eichenberger eds
Guthrie et al., 1997, J. Nutr. 127: 544-548
Henner et al., 1986, Gene 49 (1): 147-152
Horsch et al., 1985, Science 227: 1229-1231).
Hudson et al., 1984, J. Mol. Biol. 180(4): 1023-1051
Ishida et al., 1996, Nat. Biotechnol. 14(6): 745-750
Kamat et al., 1997, Mol. Cell. Biochem. 170: 131-137
Kamel-Eldin et Appelqvist, 1996, Lipids 31: 671-701
Keller et al., 1998, Eur. J. Biochem. 251(1-2): 413-417
Klein et al., 1992, Biotechnology 10(3): 286-291
Knopf, 1979, Subcell. Biochem. 6: 143-173
Kohler et al., 1995, Plant Mol. Biol., 29 (6): 1293-1298
Lawton et al., 1987, Plant Mol. Biol. 9, 315-324
Lingens et al., 1967, European J. Biochem 1: 363-374
Mannhaupt et al., 1989, Gene 85: 303-311
Martinez et al., 1989, J. Mol. Biol., 208 (4): 551-565
McPherson et al., 2000, PCR - Basics: From background to bench, First edition, Springer Verlag, Germany
Mercenier and Chassy, 1988, Biochimie 70(4): 503-517
Murashige et Skoog, 1962, Physiol. Plant. 15: 473-497
Odell et al., 1985, Nature, 313: 810-812
Packer et al., 2001, J. Nutr. 131(2): 369S-373S
Padgette et al., 1991, J. Biol. Chem. 266: 33
Pearce et al., 1992, J. Med. Chem. 35: 3595-3606
Quigley et al., 1989, J. Mol. Evol., 29 (5): 412-421
Qureshi et al., 2001, J. Nutr. 131: 2606-2618
Ralston et al., 1988, Genet., 119 (1): 185-197
Rüetschi et al., 1992, Eur. J. Biochem., 205: 459-466
Sambrook et al., 1989, Molecular Cloning: A Laboratory Manual, Second edition, Nolan C. ed., Cold Spring Harbor Laboratory Press, NY
Sambrook and Russel, 2001, Molecular cloning: A laboratory manual, Third edition, Cold Spring Harbor Laboratory Press, NY
Sampathkumar and Morrisson, 1982, Bioch Biophys Acta 702: 204-211
Sanders et al., 1987, Nucleic Acids Res. 15: 1543-1548
Schwob et al., 1993, Plant J. 4 (3): 423-432
Shaw et al., 1983, Gene 23(3): 315-330
Shigekawa and Dower, 1989, Aust. J. Biotechnol. 3(1), 56-62
Tepfer and Casse-Delbart, 1987, Microbiol. Sci. 4(1), 24-28
Viviani et al., 1998, Pestic. Biochem. Physiol. 62: 125-134
Wang et al., 1992, Mol. Cell. Biol., 12 (8): 3399-3406
White et al., 1990, Nucleic Acid Res. 18(4):1062
Xia et al., 1992, J. Gen. Microbiol. 138(7): 1309-1316

| | | |
|---|---|---|
| WO 87/07644 | US 4,945,050 | EP 0 507 698 |
| WO 91/02071 | US 5,424,276 | EP 0 508 909 |
| WO 91/13993 | US 5,994,521 | EP 0531 498 |
| WO 92/17580 | US 5,188,642 | EP 0 633 317 |
| WO 95/06128 | US 5,506,195 | EP 0 242 236 |
| WO 96/38567 | US 5,552,306 | EP 0 293 356 |
| WO 97/04103 | US 5,614,313 | EP 0 337 899 |
| WO 97/30082 | US 5,866,778 | EP 0418 175 |
| WO 97/48819 | US 5,641,876 | EP 0 470 856 |
| WO 98/22593 | | EP 0 487 352 |
| WO 98/40490 | | EP 0 496 630 |
| WO 98/45460 | | EP 0 496 631 |
| WO 98/45461 | | EP 0 527 036 |
| WO 98/46763 | | EP 0 560 482 |
| WO 98/46764 | | EP 0 625 505 |
| WO 99/02717 | | EP 0 625 508 |
| WO 99/15679 | | EP 0 682 659 |
| WO 99/16890 | | |
| WO 99/24585 | | |
| WO 99/24586 | | |
| WO 99/24594 | | |
| WO 99/34005 | | |
| WO 99/43818 | | |
| WO 99/53053 | | |
| WO 99/91089 | | |
| WO 00/01833 | | |
| WO 00/29594 | | |
| WO 00/36127 | | |

Les exemples ci-après permettent d'illustrer la présente invention, sans toutefois en limiter la portée.

Toutes les méthodes ou opérations décrites ci-dessous dans ces exemples sont données à titre d'exemples et correspondent à un choix, effectué parmi les différentes méthodes disponibles pour parvenir au même résultat. Ce choix n'a aucune incidence sur la qualité du résultat et par conséquent, toute méthode adaptée peut être utilisée par l'homme de l'art pour parvenir au même résultat. En particulier, et à moins qu'il n'en soit précisé autrement dans les exemples, toutes les techniques d'ADN recombinant employées sont mises en oeuvre selon les protocoles standards décrits dans Sambrook et al. (1989, Molecular Cloning: A Laboratory Manual, Second edition, Nolan C. ed., Cold Spring Harbor Laboratory Press, NY), dans Sambrook and Russel (2001, Molecular cloning: A laboratory manual, Third edition, Cold Spring Harbor Laboratory Press, NY), dans Ausubel et al. (1994, Current Protocols in Molecular Biology, Current protocols, USA, Volumes 1 and 2), et dans Brown (1998, Molecular Biology LabFax, Second edition, Academic Press, UK). Des matériels et méthodes standards pour la biologie moléculaire des plantes sont décrits dans Croy R.D.D. (1993, Plant Molecular Biology LabFax, BIOS Scientific Publications Ltd (UK) and Blackwell Scientific Publications (UK). Des matériels et méthodes standards pour la PCR (Polymerase Chain Reaction) sont également décrits dans Dieffenbach and Dveksler (1995, PCR Primer: A laboratory manual, Cold Spring Harbor Laboratory Press, NY) et dans McPherson et al. (2000, PCR - Basics: From background to bench, First edition, Springer Verlag, Germany).

### Exemple 1: Mesure de l'activité HPPD

L'activité HPPD peut être mesurée par la méthode décrite dans Garcia et al. (1997, Biochem. J. 325, 761-769) ou Garcia et al. (1999, Plant Physiol. 119, 1507-1516).

### Exemple 2: Mesure de l'activité préphénate deshydrogénase

L'activité préphénate deshydrogénase est mesurée à 25°C par suivi spectrophotométrique à 340 nm de la formation de NADH ou NADPH dans une solution contenant 50 mM de Tris-HCl, pH 8.6, 300 µM de préphénate, et 1 mM de NAD ou NADP dans un volume total de 200 µl.

### Exemple 3: Construction d'un gène chimère surexprimant HPPD

Un gène chimère permettant la surexpression d'HPPD pour conférer la résistance à des plantes vis-à-vis des herbicides inhibant l'HPPD a été construit.

Il consiste à assembler dans le sens de la transcription, un promoteur dit "double histone" (PdH4) tel que décrit dans la demande de brevet EP 0 507 698, la séquence du Tobacco etch virus translational enhancer (TEV) décrit dans Carrington and Freed (1990; J. Virol. 64: 1590-1597), une séquence codant pour un peptide de transit optimisé (OTP) tel que décrit dans la demande de brevet EP 0 508 909, la partie codante du gène de l'HPPD *d'Arabidopsis thaliana* décrite dans la demande de brevet WO 96/38567, puis le terminateur *nos* du gène de la nopaline synthase décrit dans Bevan et al. (1983, Nucleic Acids Res. 11(2), 369-385). L'ensemble est ensuite cloné dans un vecteur binaire et a pour structure:

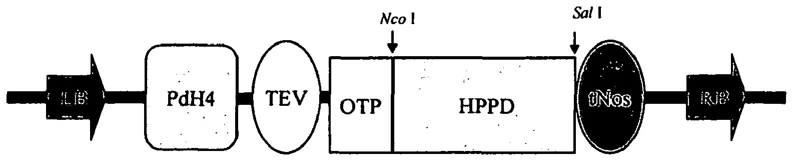
Nco I    Sal I
LB    PdH4    TEV    OTP    HPPD    tNos    RB

### Exemple 4: Construction d'un gène chimère surexprimant PDH

La construction d'un gène chimère surexprimant PDH consiste à assembler dans le sens de la transcription, un promoteur dit "double histone" (PdH4) tel que décrit dans la demande de brevet EP 0 507 698, la séquence du Tobacco etch virus translational enhancer (TEV) décrit dans Carrington and Freed (1990; J. Virol. 64: 1590-1597), une séquence codant pour un peptide de transit optimisé (OTP) tel que décrit dans la demande de brevet EP 0 508 909, la partie codante du gène PDH de levure décrite dans Mannhaupt et al. (1989, Gene 85, 303-311), puis le terminateur *nos* du gène de la nopaline synthase décrit dans Bevan et al. (1983, Nucleic Acids Res. 11(2), 369-385). L'ensemble a ensuite été cloné dans le vecteur binaire pRD 224 contenant un gène de résistance à la kanamycine (NPTII) pour donner le vecteur pRD 224-PDH. Ce vecteur a pour structure:

BamH I    Xho I
[?]B    PdH4    TEV    OTP    PDH    tNos    NPTII    RB

Ce vecteur binaire a ensuite été utilisé pour transformer la souche *d'Agrobacterium* EHA 105 et donner la souche d'*Agrobacterium* EHA 105-pRD 224-PDH. Cette souche *d'Agrobacterium* a été utilisée pour transformer du tabac PBD6 et du tabac PBD6-ARA9 (tabac transformé avec le gène chimère permettant la surexpression de l'HPPD d'*Arabidopsis thaliana*).

Les plantes transformées sont sélectionnées sur Kanamycine.

### Exemple 5: Transformation du tabac PBD6-ARA9 avec une cassette d'expression surexprimant PDH

Les tabacs PBD6-ARA9 sont des tabacs transformés avec un gène chimère tel que décrit à l'exemple 3, et surexprimant l'HPPD d'*A. thaliana* décrite dans la demande de brevet WO96/38567. La méthode d'obtention des tabacs PBD6-ARA9 est décrite dans Garcia et al. (1999, Plant Physiol. 119, 1507-1516). Les lignées PBD6-ARA9 transformées avec le gène chimère surexprimant PDH tel que décrit à l'exemple 4 sont dénommées lignées ARA9-PDH.

### 5.1: Transformation

La transformation est réalisée avec la souche non oncogène *d'Agrobacterium tumefaciens* EHA 105-pRD 224-PDH selon la technique des disques foliaires (Horsch et al., 1985, Science 227:1229-1231).

### 5.2: Régénération

La régénération du tabac ARA9-PDH est réalisée à partir d'explants foliaires sur un milieu de base Murashig et Skoog (MS) comprenant 30g/l de saccharose ainsi que 350mg/l de céfotaxime et 200 mg/ml de kanamycine. Les explants foliaires sont prélevés sur des plants en serre et régénérés selon la technique des disques foliaires (Horsch et al., 1985, Science 227: 1229-1231) en trois étapes successives :
- La première comprend l'induction des pousses sur un milieu MS additionné de 30g/l de saccharose contenant 0.05mg/l d'acide naphtylacétique (ANA) et 2mg/l de benzylaminopurine (BAP) pendant 15 jours et 200 mg/ml de kanamycine.
- Les pousses vertes formées au cours de cette étape sont ensuite développées par culture sur un milieu MS additionné de 30g/l de saccharose et 200 mg/ml de kanamycine, mais ne contenant pas d'hormone, pendant 10 jours.
- Des pousses développées sont ensuite prélevées, puis cultivées sur un milieu d'enracinement MS à teneur moitié en sels, vitamines et sucres, 200 mg/ml de kanamycine et ne contenant pas d'hormone. Au bout d'environ 15 jours, les pousses enracinées sont passées en terre.

La tolérance des plantes transformées est étudiée par semis sur un sol traité au dicétonitrile (DKN).

### Exemple 6: Tolérance des tabacs PBD6-ARA9 et ARA9-PDH aux inhibiteurs de l'HPPD

### 6.1. Tolérance au dicétonitrile (DKN)

13 lignées ARA9-PDH et la lignée PBD6-ARA9 ayant servie de matériel de départ pour la transformation ont été semées sur des concentrations croissantes de DKN: 5, 10 et 32 ppm.

A 5 ppm de DKN, toutes les lignées PBD6-ARA9 et ARA9-PDH résistent, notamment car toutes surexpriment l'HPPD d'*A*. *thaliana.* A 10 ppm de DKN, la lignée parente PBD6-ARA9 est complètement inhibée. Au contraire, toutes les lignées ARA9-PDH résistent bien, à l'exception d'une seule (ARA9-PDH4) qui est inhibée. A 32 ppm de DKN, toutes les lignées ARA9-PDH qui résistaient à 10 ppm de DKN ont des plantes qui résistent et poussent normalement, alors que la lignée parent PBD6-ARA9 n'exprimant que l'HPPD recombinante est complètement inhibée. Les lignées montrant la meilleure tolérance sont les lignées ARA9-PDH 14, ARA9-PDH 18, et ARA9-PDH24.

### 6.2. Tolérance à la sulcotrione et à la mesotrione

La même expérience a été réalisée avec les inhibiteurs de l'HPPD, sulcotrione et mesotrione. La lignée ARA9-PDH18 s'avère être tolérante à 3 µM de mesotrione et à 6 µM de sulcotrione, alors qu'une lignée de tabac sauvage du type Petit Havana est sensible à 0,375 µM de ces deux composés.

### Exemple 7: Mesure des taux de tocophérols et de tocotriénols dans des tabacs PBD6-ARA9 et ARA9-PDH

Un extrait lipidique est obtenu par la méthode de Folch (Folch et al., 1957, J. Biol. Chem., 226-497) sur des échantillons de feuilles moyennes et de très jeunes feuilles de chacune des plantes analysées. Une analyse de leurs contenus en tocophérols et tocotriénols est ensuite réalisée par HPLC selon la méthode de Frazer et al. (2000, Plant J. 24: 551-558). Ces contenus sont ensuite quantifiés par rapport à des produits de référence, puis exprimés en µg par g de masse sèche. Les résultats sont présentés dans le Tableau 1.

**Tableau 1: Taux de tocophérols et de tocotriénols dans des échantillons des plantes PBD6, PBD6-ARA9 (ARA9) et ARA9-PDH (PDH4, PDH 14, PDH 18, PDH 24).**

| Feuilles moyennes | | PBD6 | ARA9 | PDH 4 | PDH 14 | PDH 18 | PDH 24 |
|---|---|---|---|---|---|---|---|
| | | (µg par g de poids sec) | | | | | |
| α | Tocophérol | 62.2 | 64.73 | 66.9 | 89.7 | 91.03 | 83.4 |
| β/γ | Tocophérol | 1.73 | 1.93 | 2.16 | 4.56 | 4.28 | 4.01 |
| δ | Tocophérol | nd | nd | nd | nd | nd | nd |
| α | Tocotriénol | nd | nd | nd | 64.58 | 66.99 | 55.35 |
| β/γ | Tocotriénol | nd | nd | nd | 2.04 | 2.23 | 1.98 |
| δ | Tocotriénol | nd | nd | nd | nd | nd | nd |
| | | | | | | | |

| | Très jeunes feuilles | PBD6 | ARA9 | PDH 4 | PDH 14 | PDH 18 | PDH 24 |
|---|---|---|---|---|---|---|---|
| | | (µg par g de poids sec) | | | | | |
| α | Tocophérol | 73.5 | 64.73 | 68.7 | 76.2 | 75.4 | 83.4 |
| β/γ | Tocophérol | 1.83 | 2.37 | 1.86 | 3.32 | 3 | 3.5 |
| δ | Tocophérol | nd | nd | nd | nd | nd | nd |
| α | Tocotriénol | nd | nd | nd | 275.26 | 224.5 | 242.4 |
| β/γ | Tocotriénol | nd | nd | nd | 17.45 | 15.3 | 17.14 |
| δ | Tocotriénol | nd | nd | nd | 6.2 | 4.3 | 5.4 |

Ces résultats montrent clairement que les tabacs ARA9-PDH doublement transformés avec des gènes chimères permettant la sur-expression des enzymes PDH et HPPD possèdent des quantités supérieures de prénylquinones, en particulier de tocophérols et de tocotriénols, par rapport aux tabacs PBD6-ARA9 simplement transformés avec un gène codant une enzyme HPPD. L'effet le plus important concerne les tocotriénols. Cet effet est d'autant plus marqué dans les très jeunes feuilles riches en tissus méristématiques. L'origine de cette spécificité de tissu est liée au promoteur utilisé pour créer les tabacs ARA9-PDH qui est un promoteur s'exprimant préférentiellement dans les tissus en croissance rapide des plantes, en particulier les méristèmes (PdH4). L'utilisation d'autres types de promoteurs devrait permettre d'obtenir un effet similaire dans d'autres tissus de la plante.

D'autre part, les différences observées entre les différentes lignées ARA9-PDH proviennent du fait qu'il s'agit d'événements de transformation différents. Des croisements entre les meilleures lignées visant à élaborer des lignées homozygotes devraient permettre d'obtenir des lignées homogènes quant à la production de prénylquinones et à la tolérance aux inhibiteurs d'HPPD.

## Revendications

1. Plantes transformées, **caractérisées en ce qu'**elles comprennent:
(1) un gène chimère comprenant un promoteur fonctionnel dans les plantes et une séquence codant pour une enzyme Préphénate Deshydrogénase (PDH), à l'exception de la séquence codante du gène TyrA *d'Erwinia herbicola*
(2) un gène chimère comprenant un promoteur fonctionnel dans les plantes et une séquence codant pour une enzyme p-hydroxyphenylpyruvate dioxygénase (HPPD).

2. Cellules végétales transformées, **caractérisées en ce qu'**elles comprennent:
(1) un gène chimère comprenant un promoteur fonctionnel dans les plantes et une séquence codant pour une enzyme PDH, à l'exception de la séquence codante du gène TyrA *d'Erwinia herbicola*
(2) un gène chimère comprenant un promoteur fonctionnel dans les plantes et une séquence codant pour une enzyme HPPD.

3. Plantes selon la revendication 1, ou cellules végétales selon la revendication 2, **caractérisées en ce que** la séquence codant pour une enzyme PDH est la séquence codante d'un gène codant pour une PDH de levure.

4. Plantes ou cellules végétales selon la revendication 3, **caractérisées en ce que** la séquence codante d'un gène codant pour une PDH de levure est la séquence codante d'un gène de *saccharomyces cereviseae.*

5. Plantes ou cellules végétales selon l'une des revendications 1 à 4, **caractérisées en ce que** la séquence codant pour une enzyme HPPD est la séquence codante d'un gène codant pour une HPPD de plante.

6. Plantes ou cellules végétales selon la revendication 5, **caractérisées en ce que** la séquence codante d'un gène codant pour une HPPD de plante est la séquence codante d'un gène d'Arabidopsis *thaliana.*

7. Procédé de culture des plantes transformées selon l'une des revendications 1 à 6, **caractérisé en ce qu'**il consiste à planter les graines desdites plantes transformées sur une surface d'un champ approprié pour la culture desdites plantes, à appliquer sur ladite surface dudit champ au moins une composition herbicide comprenant un inhibiteur d'HPPD, puis à récolter les plantes cultivées lorsqu'elles arrivent à la maturité souhaitée et éventuellement à séparer les graines des plantes récoltées.

8. Procédé pour conférer aux plantes une tolérance aux inhibiteurs d'HPPD, **caractérisé en ce que** l'on transforme lesdites plantes, simultanément ou successivement, avec:
(1) un gène chimère comprenant un promoteur fonctionnel dans les plantes et une séquence codant pour une enzyme PDH
(2) un gène chimère comprenant un promoteur fonctionnel dans les plantes et une séquence codant pour une enzyme HPPD.

9. Procédé pour augmenter la quantité de prénylquinones dans les plantes, **caractérisé en ce que** l'on transforme lesdites plantes, simultanément ou successivement, avec:
(1) un gène chimère comprenant un promoteur fonctionnel dans les plantes et une séquence codant pour une enzyme PDH, à l'exception de la séquence codante du gène TyrA *d'Erwinia herbicola*
(2) un gène chimère comprenant un promoteur fonctionnel dans les plantes et une séquence codant pour une enzyme HPPD.

10. Procédé de production de prénylquinones, **caractérisé en ce qu'**il comprend une étape de mise en culture d'une cellule végétale ou d'une plante transformée selon l'une des revendications 1 à 6 dans un milieu de culture adapté à la croissance et à la multiplication de ladite cellule végétale ou de ladite plante.

11. Procédé selon l'une des revendications 9 ou 10, **caractérisé en ce que** les prénylquinones sont des tocotriénols.

12. Procédé selon l'une des revendications 9 ou 10, **caractérisé en ce que** les prénylquinones sont représentés par la vitamine E.

13. Plantes ou cellules végétales selon l'une des revendications 1 à 6, **caractérisées en ce qu'**elles comprennent, en plus, un gène chimère comprenant un promoteur fonctionnel dans les plantes et une séquence codant pour une enzyme géranylgéranyl réductase (GGR).

14. Procédé selon l'une des revendications 8 ou 9, **caractérisé en ce que** l'on transforme lesdites plantes, simultanément ou successivement, avec en plus un gène chimère comprenant un promoteur fonctionnel dans les plantes et une séquence codant pour une enzyme GGR.

## Claims

1. Transformed plants, **characterized in that** they comprise:
(1) a chimeric gene comprising a promoter that is functional in plants and a sequence encoding a prephenate dehydrogenase (PDH) enzyme, with the exception of the coding sequence of the TyrA gene of *Erwinia herbicola;*
(2) a chimeric gene comprising a promoter that is functional in plants and a sequence encoding a p-hydroxyphenylpyruvate dioxygenase (HPPD) enzyme.

2. Transformed plant cells, **characterized in that** they comprise:
(1) a chimeric gene comprising a promoter that is functional in plants and a sequence encoding a PDH enzyme, with the exception of the coding sequence of the TyrA gene of *Erwinia herbicola;*
(2) a chimeric gene comprising a promoter that is functional in plants and a sequence encoding an HPPD enzyme.

3. Plants according to Claim 1, or plant cells according to Claim 2, **characterized in that** the sequence encoding a PDH enzyme is the coding sequence of a gene encoding a yeast PDH.

4. Plants or plant cells according to Claim 3, **characterized in that** the coding sequence of a gene encoding a yeast PDH is the coding sequence of a *Saccharomyces cerevisiae* gene.

5. Plants or plant cells according to one of Claims 1 to 4, **characterized in that** the sequence encoding an HPPD enzyme is the coding sequence of a gene encoding a plant HPPD.

6. Plant or plant cells according to Claim 5, **characterized in that** the coding sequence of a gene encoding a plant HPPD is the coding sequence of an *Arabidopsis thaliana* gene.

7. Method for cultivating the transformed plants according to one of Claims 1 to 6, **characterized in that** it consists in planting the seeds of said transformed plants over an area of a field that is suitable for the cultivation of said plants, in applying to said area of said field at least one herbicidal composition comprising an HPPD inhibitor, and then in harvesting the cultivated plants when they reach the desired maturity and, optionally, in separating the seeds from the harvested plants.

8. Method for conferring on plants a tolerance to HPPD inhibitors, **characterized in that** said plants are transformed, simultaneously or successively, with:
(1) a chimeric gene comprising a promoter that is functional in plants and a sequence encoding a PDH enzyme;
(2) a chimeric gene comprising a promoter that is functional in plants and a sequence encoding an HPPD enzyme.

9. Method for increasing the amount of prenylquinones in plants, **characterized in that** said plants are transformed, simultaneously or successively, with:
(1) a chimeric gene comprising a promoter that is functional in plants and a sequence encoding a PDH enzyme, with the exception of the coding sequence of the TyrA gene of *Erwinia herbicola;*
(2) a chimeric gene comprising a promoter that is functional in plants and a sequence encoding an HPPD enzyme.

10. Method for producing prenylquinones, **characterized in that** it comprises a step of cultivating a transformed plant cell or plant according to one of Claims 1 to 6 in a cultivation medium that is suitable for the growth and for the multiplication of said plant cell or of said plant.

11. Method according to either of Claims 9 and 10, **characterized in that** the prenylquinones are tocotrienols.

12. Method according to either of Claims 9 and 10, **characterized in that** the prenylquinones are represented by vitamin E.

13. Plants or plant cells according to one of Claims 1 to 6, **characterized in that** they comprise, in addition, a chimeric gene comprising a promoter that is functional in plants and a sequence encoding a geranylgeranyl reductase (GGR) enzyme.

14. Method according to either of Claims 8 and 9, **characterized in that** said plants are transformed, simultaneously or successively, with, in addition, a chimeric gene comprising a promoter that is functional in plants and a sequence encoding a GGR enzyme.

## Patentansprüche

1. Transformierte Pflanzen, **dadurch gekennzeichnet, dass** sie Folgendes umfassen:
(1) ein chimäres Gen, umfassend einen in Pflanzen operativen Promoter und eine Sequenz, die für eine Präphenatdehydrogenase (PDH) codiert, mit Ausnahme der für das Gen TyrA aus *Erwinia herbicola* codierenden Sequenz,
(2) ein chimäres Gen, umfassend einen in Pflanzen operativen Promoter und eine Sequenz, die für eine p-Hydroxyphenylpyruvatdioxygenase (HPPD) codiert.

2. Transformierte Pflanzenzellen, **dadurch gekennzeichnet, dass** sie Folgendes umfassen:
(1) ein chimäres Gen, umfassend einen in Pflanzen operativen Promoter und eine Sequenz, die für eine PDH codiert, mit Ausnahme der für das Gen TyrA aus *Erwinia herbicola* codierenden Sequenz,
(2) ein chimäres Gen, umfassend einen in Pflanzen operativen Promoter und eine Sequenz, die für eine HPPD codiert.

3. Pflanzen nach Anspruch 1 oder Pflanzenzellen nach Anspruch 2, **dadurch gekennzeichnet, dass** es sich bei der Codiersequenz für eine PDH um die Codiersequenz eines Gens, das für eine Hefe-PDH codiert, handelt.

4. Pflanzen oder Pflanzenzellen nach Anspruch 3, **dadurch gekennzeichnet, dass** es sich bei der Codiersequenz eines Gens, das für eine Hefe-PDH codiert, um die Codiersequenz eines *Saccharomyces-cerevisiae-*Gens handelt.

5. Pflanzen oder Pflanzenzellen nach einem der Ansprüche 1 bis 4, **dadurch gekennzeichnet, dass** es sich bei der Codiersequenz für eine HPPD um die Codiersequenz eines Gens, das für eine Pflanzen-HPPD codiert, handelt.

6. Pflanzen oder Pflanzenzellen nach Anspruch 5, **dadurch gekennzeichnet, dass** es sich bei der Codiersequenz eines Gens, das für eine Hefe-HPPD codiert, um die Codiersequenz eines *Arabidopsis thaliana-*Gens handelt.

7. Verfahren zum Kultivieren von transformierten Pflanzen nach einem der Ansprüche 1 bis 6, **dadurch gekennzeichnet, dass** es darin besteht, dass man die Samen der transformierten Pflanzen auf der Oberfläche eines für die Kultur dieser Pflanzen geeigneten Feldes anbaut, auf die Oberfläche des Feldes mindestens eine herbizide Verbindung, umfassend einen HPPD-Hemmer, ausbringt und anschließend die kultivierten Pflanzen, wenn sie die gewünschte Reife erreicht haben, erntet und gegebenenfalls die Samen von den geernteten Pflanzen abtrennt.

8. Verfahren zum Verleihen einer HPPD-Hemmer-Toleranz an Pflanzen, **dadurch gekennzeichnet, dass** man die Pflanzen gleichzeitig oder der Reihe nach mit Folgendem transformiert:
(1) einem chimären Gen, umfassend einen in Pflanzen operativen Promoter und eine Codiersequenz für eine PDH,
(2) einem chimären Gen, umfassend einen in Pflanzen operativen Promoter und eine Codiersequenz für eine HPPD.

9. Verfahren zum Erhöhen der Prenylchinonmenge in Pflanzen, **dadurch gekennzeichnet, dass** man die Pflanzen gleichzeitig oder der Reihe nach mit Folgendem transformiert:
(1) einem chimären Gen, umfassend einen in Pflanzen operativen Promoter und eine Codiersequenz für eine PDH, mit Ausnahme der für das Gen TyrA aus *Erwinia herbicola* codierenden Sequenz,
(2) einem chimären Gen, umfassend einen in Pflanzen operativen Promoter und eine Codiersequenz für eine HPPD.

10. Verfahren zur Herstellung von Prenylchinonen, **dadurch gekennzeichnet, dass** es einen Schritt des Züchtens einer Pflanzenzelle oder einer transformierten Pflanze nach einem der Ansprüche 1 bis 6 in einem Kulturmedium, das sich für das Wachstum und die Vermehrung der Pflanzenzelle oder der Pflanze eignet.

11. Verfahren nach einem der Ansprüche 9 oder 10, **dadurch gekennzeichnet, dass** es sich bei den Prenylchinonen um Tocotrienole handelt.

12. Verfahren nach einem der Ansprüche 9 oder 10, **dadurch gekennzeichnet, dass** es sich bei den Prenylchinonen um Vitamin E handelt.

13. Pflanzen oder Pflanzenzellen nach einem der Ansprüche 1 bis 6, **dadurch gekennzeichnet, dass** sie weiterhin ein chimäres Gen umfassend einen in Pflanzen operativen Promoter und eine Codiersequenz für eine Geranylgeranylreductase (GGR) umfassen.

14. Verfahren nach einem der Ansprüche 8 oder 9, **dadurch gekennzeichnet, dass** man die Pflanzen gleichzeitig oder der Reihe nach zusätzlich mit einem chimären Gen, umfassend einen in Pflanzen operativen Promoter und eine Codiersequenz für eine GGR, transformiert.
